# EUROPEAN PATENT SPECIFICATION

(11) **EP 2 123 666 B1**
(45) Date of publication and mention of the grant of the patent: **01.05.2013**
(21) Application number: 08721044.9
(22) Date of filing: 29.02.2008
(51) Int. Cl.: C07K 7/08, A23K 1/16, A23L 1/305, A61K 38/00, A61P 1/16, C07K 5/09, C07K 5/11, C07K 7/06, C07K 14/47

(54) **Peptides from casein for use as agents in liver pathologies**
Peptide aus Casein zur Verwendung als Wirkstoffe in Leberkrankheiten
Peptides dérivés de la caséïne en tant qu'agents thérapeutiques dans des pathologies hépatiques

(30) Priority: 02.03.2007 JP 2007052520
(43) Date of publication of application: 25.11.2009
(73) Proprietor: Megmilk Snow Brand Co., Ltd., Sapporo-shi Hokkaido 0650043 (JP)
(72) Inventor: KAWAKAMI, Hiroshi, Kawagoe-shi Saitama 350-1142 (JP); KADOOKA, Yukio, Kawagoe-shi Saitama 350-1165 (JP); HOSOYA, Tomohiro, Kawagoe-shi Saitama 350-1165 (JP); SATO, Kenji, Kyoto-shi Kyoto 603-8206 (JP)
(74) Representative: Høiberg A/S
(86) International application number: PCT/JP2008/053596
(87) International publication number: WO 2008/108285

(56) References cited:
- EP-A1- 2 017 283
- WO-A1-2005/028512
- WO-A2-2005/081628
- JP-A- 2006 131 626
- JP-A- 2007 254 448
- JP-A- 2007 254 449
- US-A1- 2005 281 914
- J A GOMEZ-RUIZ ET AL.: "Sensory and mass spectrometric analysis of the peptidic fraction lower than one thousand daltons in Manchego cheese", JOURNAL OF DAIRY RESEARCH, vol. 90, no. 11, November 2007 (2007-11), pages 4966-4973, XP26956245, CAMBRIDGE UNIVERSITY PRESS, CAMBRIDGE ISSN: 0022-0299
- A QUIROS ET AL.: "Angiotensin-converting enzyme inhibitory activity of peptides derived from caprine Kefir", JOURNAL OF DAIRY RESEARCH, vol. 88, no. 10, October 2005 (2005-10), pages 3480-3487, XP2443592, CAMBRIDGE UNIVERSITY PRESS, CAMBRIDGE ISSN: 0022-0299
- M SCHURINK ET AL.: "Improvement of lipoxygenase inhibition by octapeptides", PEPTIDES, vol. 28, no. 12, December 2007 (2007-12), pages 2268-2275, XP22350463, USELSEVIER, AMSTERDAM ISSN: 0196-9781
- LEMIEUX L. ET AL.: 'Application of reversed-phase high-performance liquid chromatography to the separation of peptides from phosphorylated and dephosphorylated casein hydrolyzates' JOURNAL OF CHROMATOGRAPHY vol. 473, no. 1, 1989, pages 189 - 206, XP009070046
- TONOUCHI H. ET AL.: 'Concanavalin A Yuhatsu Kanshogai Model Mouse ni Okeru Kakushu Cheese no Koka' THE JAPANESE SOCIETY OF NUTRITION AND FOOD SCIENCE TAIKAI KOEN YOSHISHU vol. 60TH, 2006, page 267 + ABSTR. NO. 3B-13P, XP003016086
- SAITO T. ET AL.: 'Isolation and structural analysis of antihypertensive peptides that exist naturally in Gouda Cheese' JOURNAL OF DAIRY SCIENCE vol. 83, no. 7, 2000, pages 1434 - 1440, XP002944963

## Description

### TECHNICAL FIELD

The present invention relates to a peptide having a hepatic dysfunction-inhibitory effect. Further, the present invention relates to a peptide being derived from a milk protein and having a hepatic dysfunction-inhibitory effect. Still further, the present invention relates to a hepatic dysfunction inhibitor containing the peptide as an active ingredient. Yet still further, the present invention relates to a food, beverage or feed containing the peptide. A hepatic dysfunction inhibitor and a food or beverage for inhibiting (suppressing) hepatic dysfunction of the present invention are useful for the treatment and prevention of diseases due to hepatic dysfunction, because they may be ingested to inhibit (abrogate) hepatic dysfunction such as alcoholic hepatic dysfunction.

### BACKGROUND ART

The liver is an important organ which is responsible for various functions such as metabolism and storage of a carbohydrate, a protein, a lipid, and the like, or degradation and detoxification of harmful substances. Such liver functions suffer dysfunction by various factors such as viral infection, stresses, smoking, unhealthy dietary habits, and drinking. As a result, there is an increased risk of getting diseases such as acute hepatitis, chronic hepatitis, viral hepatitis, alcoholic fatty liver, hepatic cirrhosis, and liver cancer. In particular, the increase in the amount of alcohol ingestion in dietary habit increases a risk of developing hepatic dysfunction.

It should be noted that when the liver function suffers dysfunction due to various factors, enzymes such as aspartate aminotransferase (which is also referred to as glutamate oxaloacetate transaminase, and abbreviated as GOT) and alanine aminotransferase (which is also referred to as glutamate pyruvate transaminase, and abbreviated as GPT), each of which is present in hepatocytes, leak into blood, and hence, both of blood GOT activity and blood GPT activity are indicators of hepatic dysfunction.

Hepatic dysfunction may be treated with pharmaceuticals. However, there is a consensus that hepatic dysfunction should be desirably minimized by a preventative action. From the viewpoint of the foregoing, the most suitable means for the preventative action is a food and a food ingredient, each of which may be ingested on a daily basis. Then, as a food for inhibiting hepatic dysfunction, there are known turmeric, an oyster extract, a lever extract, and the like.

In recent years, researches on a physiological function of a food and a food ingredient have been extensively carried out. Also for cheese, it has been reported that, in spite of being a high-fat-containing food, cheese has an effect of promoting the reduction in blood triglyceride concentration and an effect of improving cholesterol metabolism (Patent Document 1 and Patent Document 2). Further, it has been reported that cheese also has an effect of inhibiting oxidative damage caused by oxygen radical, a free radical, and the like, each of which exerts a negative impact on diseases, senescence, and the like in a living body (Patent Document 3).
Patent Document 1: JP 2003-300890 A
Patent Document 2: JP 2003-144090 A
Patent Document 3: JP 2004-352958 A

EP 12017283 discloses the peptides of the present application having SEQ ID NO:s 12 and 15, but does not disclose use of said peptides for the treatment of a disease which is due to hepatic dysfunction wherein the disease is selected from the group consisting of acute hepatitis, chronic hepatitis, viral hepatitis, alcoholic fatty liver, hepatic cirrhosis, and liver cancer.

### DISCLOSURE OF THE INVENTION

### PROBLEM TO BE SOLVED BY THE INVENTION

An object of the present invention is to provide a hepatic dysfunction inhibitor or a food or beverage for inhibiting hepatic dysfunction containing a peptide contained in cheese as an active ingredient, which is useful for the inhibition of hepatic dysfunction through its ingestion, and may be ingested on a daily basis.

### MEANS FOR SOLVING THE PROBLEM

The inventors of the present invention have intensively studied in order to solve the above-mentioned problem in terms of preventing and improving various dysfunctions in a living body by a food or a food ingredient which may be ingested on a daily basis. As a result, the inventors have found that a peptide which includes any one of amino acid sequences represented by the following formulae (1) to (28) has a hepatic dysfunction-inhibitory effect, and has the effect at a lower dose, and thus have completed the present invention.
(1) Arg-Pro-Lys-His-Pro-Ile-Lys-His-Gln-Gly-Leu-Pro-Gln-Glu
(2) His-Ile-Gln-Lys-Glu-Asp-Val-Pro-Ser-Glu-Arg-Tyr-Leu-Gly-Tyr-Leu-Glu-Gln-Leu-Leu-Arg-Leu-Lys-Lys-Tyr-Lys-Val-Pro-Gln-Leu
(3) Ile-Asn-Pro-Ser-Lys-Glu-Asn
(4) Gln-His-Gln-Lys-Ala-Met-Lys-Pro
(5) Lys-Phe-Gln-Ser-Glu-Glu-Gln
(6) Asp-Lys-Ile-His-Pro-Phe
(7) Ile-Pro-Pro-Leu-Thr-Gln-Thr-Pro-Val-Val-Val-Pro-Pro-Phe-Leu-Gln-Pro-Glu-Val-Met-Gly-Val-Ser-Lys-Val-Lys-Glu-Ala-Met-Ala-Pro-Lys-Gln-Lys-Glu-Met
(8) Tyr-Gln-Glu-Pro-Val-Leu-Gly-Pro-Val-Arg
(9) pyroGlu-Glu-Lys-Asn-Met (pyroGlu: pyroglutamate)
(10) Arg-Pro-Lys-His
(11) Arg-Pro-Lys-His-Pro-Ile-Lys-His-Gln-Gly-Leu
(12) His-Pro-Ile-Lys
(13) His-Pro-Ile-Lys-His-Gln
(14) His-Pro-Ile-Lys-His-Gln-Gly-Leu-Pro
(15) His-Pro-Ile-Lys-His-Gln-Gly-Leu-Pro-Gln
(16) Val-Ala-Pro-Phe-Pro
(17) Lys-Val-Asn
(18) His-Ile-Gln-Lys-Glu-Asp-Val-Pro-Ser-Glu-Arg-Tyr
(19) Asp-Val-Pro-Ser-Glu-Arg-Tyr-Leu-Gly-Tyr-Leu-Glu-Gln-Leu-Leu-Arg-Leu-Lys-Lys-Tyr-Lys-Val-Pro-Gln-Leu
(20) Ile-Asn-Pro-Ser
(21) Lys-Phe-Gln-Ser-Glu
(22) Phe-Gln-Ser-Glu
(23) Phe-Gln-Ser-Glu-Glu-Gln
(24) Ile-Pro-Pro-Leu-Thr-Gln-Thr-Pro-Val-Val-Val
(25) Leu-Thr-Gln-Thr-Pro-Val-Val-Val-Pro-Pro-Phe-Leu-Gln-Pro
(26) Thr-Gln-Thr-Pro-Val-Val-Val-Pro-Pro-Phe-Leu-Gln-Pro-Glu-Val-Met
(27) Gly-Val-Ser-Lys-Val-Lys-Glu-Ala-Met-Ala
(28) Leu-Leu-Tyr-Gln-Glu-Pro-Val-Leu-Gly-Pro-Val-Arg-Gly-Pro-Phe-Pro-Ile

That is, the present invention relates to a peptide having a hepatic dysfunction-inhibitory effect, which is represented by any one of the above formulae (1) to (28), provided that serine (Ser) may be phosphorylated.

Further, the present invention relates to a peptide being derived from a milk protein and having a hepatic dysfunction-inhibitory effect, which includes any one of amino acid sequences represented by any one of the above formulae (1) to (28).

Still further, the present invention relates to a hepatic dysfunction inhibitor containing a peptide represented by any one of the above formulae (1) to (28) as an active ingredient.

Yet still further, the present invention relates to a food, beverage or feed for inhibiting hepatic dysfunction containing a peptide represented by any one of the above formulae (1) to (28).

### EFFECTS OF THE INVENTION

The peptide having a hepatic dysfunction-inhibitory effect, which includes any one of amino acid sequences represented by the formulae (1) to (28), of the present invention may be ingested to inhibit (abrogate) hepatic dysfunction, and hence is useful for the prevention and improvement of diseases such as acute hepatitis, chronic hepatitis, viral hepatitis, alcoholic fatty liver, hepatic cirrhosis, and liver cancer. Therefore, the peptide may be used as a hepatic dysfunction inhibitor containing the peptide as an active ingredient and a food, beverage or feed for inhibiting hepatic dysfunction containing the peptide.

### BRIEF DESCRIPTION OF THE DRAWINGS

[Figure 1] is a graph illustrating an average value of blood GOT activity in each experiment group (Reference Example 1).
[Figure 2] is a graph illustrating an average value of blood GPT activity in each experiment group (Reference Example 1).
[Figure 3] is a chart showing a chromatographic pattern and a fraction A (Test Example 1).
[Figure 4] is a chart showing a chromatographic pattern and a fraction B (Test Example 1).
[Figure 5] is a graph illustrating an average value of blood GOT activity in each experiment group (Test Example 1).
[Figure 6] is a graph illustrating an average value of blood GPT activity in each experiment group (Test Example 1).

### BEST MODE FOR CARRYING OUT THE INVENTION

A peptide having a hepatic dysfunction-inhibitory effect, which includes any one of amino acid sequences represented by the formulae (1) to (28) that can be used in the present invention may be obtained by, for example, suspending cheese into a solvent, and thereafter defatting the suspension and removing an insoluble substance by centrifugation. In addition, a protein may be removed from the resulting fraction. In the present invention, the phrase "suspending cheese into a solvent" refers to adding a solvent to cheese and homogenizing the mixture, or crushing the cheese in the solvent, to thereby yield the cheese having a size that allows a water-soluble peptide fraction to be easily obtained. As the solvent, there may be used an aqueous solvent such as water or a phosphate buffer. After that, desalting may be performed with a dialysis membrane, an ion exchange resin, or the like. In addition, drying such as lyophilization or spray drying may be performed to form powders. Further, there may also be used fractions obtained by purifying those fractions with a dialysis membrane and a variety of chromatography such as gel filtration chromatography and ion exchange chromatography.

Further, as a cheese raw material for obtaining the peptide having a hepatic dysfunction-inhibitory effect, which includes any one of amino acid sequences represented by the formulae (1) to (28), for example, there may be used natural cheese such as Parmesan cheese, Gruyere cheese, Marivaux cheese, Gouda cheese, Cheddar cheese, Emmental cheese, Edam cheese, Camembert cheese, Brie cheese, Munster cheese, Pont-l'Évêque cheese, Stilton cheese, Danablu cheese, or blue cheese, and process cheese using the above-mentioned natural cheese as a raw material. In particular, it is desired to use highly aged natural cheese.

In addition, the fraction containing a peptide which includes any one of amino acid sequences represented by the formulae (1) to (28), which is obtained by suspending cheese into a solvent, and thereafter, defatting the suspension, removing an insoluble substance, and removing an undegraded protein, may be further purified by reverse phase chromatography by using a C18 column. When the fraction containing a peptide is passed through the C18 column under an acidic condition (e.g., trifluoroacetic acid (TFA)) or under a neutral condition (e.g., distilled water), fractions having a hepatic dysfunction-inhibitory effect are mainly divided into a permeable fraction which is not adsorbed to the column, and a fraction which is adsorbed to the column and eluted with 10% ethanol. When those fractions are further purified by gel filtration chromatography, active fractions have molecular weight distribution ranging from 400 to 6,000 in either fractions.
In addition, the fraction containing a peptide is dissolved with 0.05% TFA, and then subjected to reverse phase HPLC by using a YMC-Pack ODS-A column (4.6 mm× 150 mm), to thereby fractionate the peptide. Chromatography is desirably carried out with the following conditions: a solvent (solution A: 0.05% TFA; solution B: 100% acetonitrile); a concentration gradient (from 0% B to 45% B, 125 min); a flow rate: 0.8 ml/min; and a detection wavelength: 220 nm.

By the way, maturity (%) which is an indicator of the degree to which cheese ages is calculated with the following equation: [(soluble nitrogen/total nitrogen)×100]. The maturity is about 6 to 7% immediately after the manufacture of natural cheese. However, in some cheese, the maturity rises up to about 24 to 30% along with aging. In the present invention, it has been confirmed that when the maturity becomes higher as cheese ages, a higher hepatic dysfunction-inhibitory effect is obtained. This means that a peptide component effective for a hepatic dysfunction-inhibitory effect increases as cheese ages. Thus, in the present invention, it is desired to use a peptide component which increases as cheese ages, as a peptide component contained in cheese, . There may also be used cheese itself containing those peptide components, or a concentrate of cheese containing those peptide components.

A peptide having a hepatic dysfunction-inhibitory effect which includes any one of amino acid sequences represented by the formulae (1) to (28) of the present invention and a fraction containing the peptide may be orally or parenterally administered to inhibit (abrogate) hepatic dysfunction or the like in a living body, and hence may treat and prevent diseases due to hepatic dysfunction. In case of administering orally or parenterally, as a dosage form of the peptide having a hepatic dysfunction-inhibitory effect of the present invention, there may be exemplified formulations such as tablets, capsules, granules, powdered formulations, powders, and syrups. Further, examples of the food or beverage for inhibiting hepatic dysfunction include bread, a snack, goodies, a cake, a pudding, a beverage, fermented milk, noodles, a sausage, and various powdered milk and weaning diets.

The oral dosage of the peptide having a hepatic dysfunction-inhibitory effect which includes any one of amino acid sequences represented by the formulae (1) to (28) of the present invention may be appropriately determined taking into consideration the purpose of treatment and prevention, as well as symptoms, body weight, age, gender, and the like. Generally, when the peptide which includes any one of amino acid sequences represented by the formulae (1) to (28) is administered in an amount of 10 to 1,000 mg per day for each adult human, an effect of treating and preventing diseases due to hepatic dysfunction is obtained. Thus, the present invention has an effect even at a lower dose.

Hereinafter, the invention is explained in more detail by way of examples and test examples. Those examples are only illustrative, and the present invention is in no way limited thereto.

### EXAMPLE 1

### (Preparation of peptide of Gouda-type cheese)

A raw material milk was subjected to heat sterilization (75°C, 15 seconds) and then cooled to 30°C, followed by addition of 0.01 % calcium chloride. In addition, 0.7% of a commercially-available lactobacillus starter (LD starter, Chr. Hansen A/S) and 1% of Lactobacillus helveticus (L. helveticus) SBT2171 (FERMP-14381), which is one of polysaccharide-producing lactobacilli, were added, and 0.003% of rennet was further added to coagulate the milk. The thus obtained curd was cut and stirred until pH became 6.2 to 6.1, followed by discharge of whey, to thereby afford curd granules. Then, the curd granules were filled into a mold and squeezed, and further salted. The resultant was aged at 10°C for 8 months to prepare hard-type natural cheese (Gouda-type cheese).

To 20 g of the prepared Gouda-type cheese was added 80 ml of distilled water, followed by grinding with Stomacher (ORGANO Corporation) for 15 minutes. After that, the resultant was further crushed with a ultradisperser (ULTRA-TURRAX, T-25; IKA Japan K.K.) in water for 30 seconds. Milk fat generated at the time of homogenization was removed. The resulting cheese slurry was shaken with a shaker for 30 minutes, and then centrifuged (6,000 rpm, 20 min, 4°C) to remove insoluble matters, and the supernatant was filtered with a paper filter (No. 113; Whatman Ltd.). To the resulting filtrate was added ethanol so as to achieve a concentration of 70%, and the whole was left to stand still at 4°C for 4 hours and then centrifuged (10,000 rpm, 20 min, 4°C) to remove insoluble matters. After removal of ethanol with an evaporator, lyophilization was performed to afford a water-soluble peptide fraction of Gouda-type cheese.

The water-soluble peptide fraction was dissolved with 0.05% TFA, and then subjected to reverse phase HPLC with a YMC-Pack ODS-A column (4.6 mm× 150 mm) to fractionate a water-soluble peptide purified fraction. The chromatography was carried out with the following conditions: solvent (solution A: 0.05% TFA; solution B: 100% acetonitrile); concentration gradient (from 0% B to 45% B, 125 min); flow rate: 0.8 ml/min; and detection wavelength: 220 nm.

The water-soluble peptide purified fraction was subjected to reverse phase HPLC by using Cosmosil 5C22-AR-II (4.6 mm× 150 mm) and further fractionated to afford a peptide. The chromatography was carried out with the following conditions: solvent (solution A: 0.1% TFA; solution B: 80% acetonitrile); concentration gradient (from 0% B to 15% B, 40 min); flow rate: 0.8 ml/min; and detection wavelength: 220 nm.

The thus obtained peptide was analyzed for its amino acid sequence with a peptide sequencer (Applied Biosystems, Inc.). It should be noted that among amino acids, pyroglutamate generated by intramolecular binding of an α-amino group with a γ-carboxyl group in the glutamic acid was analyzed by a pre-labeling method using 2-nitrophenylhydrazine. The results confirmed a peptide which includes any one of amino acid sequences represented by the formulae (1) to (28). When the resulting peptide was measured for a hepatic dysfunction-inhibitory effect by a method according to Test Example 1 mentioned below, it was confirmed to have a high hepatic dysfunction-inhibitory effect.

Those peptides are rarely observed in unaged cheese, but are generated through aging. Further, the thus obtained peptide of the present invention may be directly used as a hepatic dysfunction inhibitor.

### [Reference Example 1]

### (Confirmation of hepatic dysfunction-inhibitory effect by aged cheese)

The 5-month-aged cheese (maturity: 30%) and 8-month-aged cheese (maturity: 38%) manufactured in Example 1 were used for the purpose of confirming an effect of inhibiting hepatic dysfunction caused by alcohol. Further, unaged cheese was used as a control. An animal experiment was performed by dividing animals into a 5-month-aged cheese-administered group (5-month-aged group), an 8-month-aged cheese-administered group (8-month-aged group), and an unaged cheese-administered group (unaged group), each of which had 5 animals.

The animal experiment was performed in accordance with a liquid feed method (Lin, Liquid diet preparation for study of chronic alcohol ingestion in the rat, Lab. Anim. Sci., vol. 39, p. 618-620, 1989). That is, a liquid feed containing 20% of each cheese, 8.5% of sucrose, and 5% of ethanol was prepared and administered to C57BL/6 mice (5-week-old, male, Charles river laboratories Japan, Inc.). One week after administration, the activities of GOT and GPT, which leaked from hepatocytes that suffered dysfunction into blood, were measured with a FUJI DRI-CHEM FDC5500 system. It should be noted that the ingestion amount of the liquid feed was 8.8 g±0.4 g per mouse per day on average, and there was no difference between the groups.

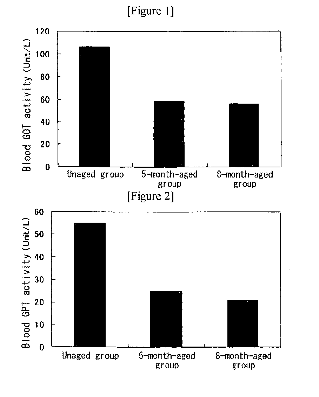

The results are shown in FIG. 1 and FIG. 2. The results indicate that GOT activity and GPT activity showed significantly lower values in both the 5-month-aged group and the 8-month-aged group, compared with the unaged group. In other words, it was found that hepatic dysfunction might be reduced by ingesting a peptide component which increases as cheese ages.

### [Test Example 1]

### (Confirmation of hepatic dysfunction-inhibitory effect by using water-soluble peptide fraction)

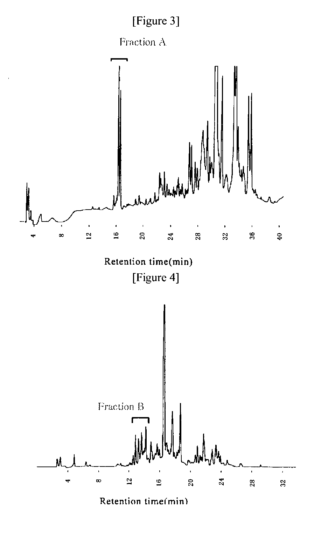

FIG. 3 and FIG. 4 each shows a typical chromatographic pattern of a fraction obtained by further fractionating the water-soluble peptide fraction in Example 1. In Test Example 1, a fraction A in FIG. 3 and a fraction B in FIG. 4 were each collected to confirm an effect of inhibiting hepatic dysfunction caused by alcohol in those fractions. Meanwhile, confirmation has been made from an amino acid sequence analysis that the fraction A contains peptides which include amino acid sequences represented by the formulae (10), (11), (15), and (27), and the fraction B contains peptides which include amino acid sequences represented by the formulae (9), (20), (21), and (23).

A liquid feed containing 20% of unaged cheese, 8.5% of sucrose, 5% of ethanol, and 0.1% of a lyophilizate of the fraction A or B was prepared to evaluate the degree of hepatic dysfunction in the same manner as in Reference Example 1.

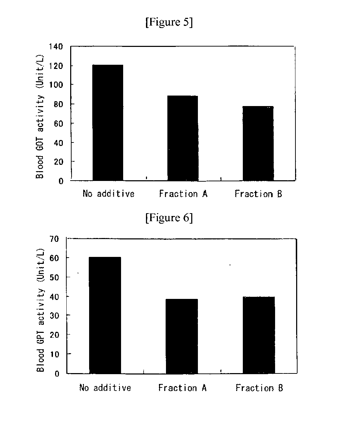

The results are shown in FIG. 5 and FIG. 6. The results indicate that a group to which the fraction was added exhibited significantly lower values in GOT activity and GPT activity, each of which was a hepatic dysfunction marker, compared with a group to which the fraction was not added.

It is clear from Reference Example 1 mentioned above that unaged cheese exhibited a lower hepatic dysfunction-inhibitory effect, which dysfunction is caused by alcohol, compared with aged cheese. On the other hand, when the fraction containing a peptide was added to unaged cheese, a hepatic dysfunction-inhibitory effect was exhibited. Thus, it was confirmed that the peptide fraction exhibited the hepatic dysfunction-inhibitory effect.

### [Test Example 2]

### (Measurement of cellular cytotoxicity-inhibitory activity of peptide)

A test for cellular injury with a human-derived hepatocyte strain (Hep G2, ATCC HB8065) was performed by using the peptide which includes any one of amino acid sequences represented by the formulae (1) to (28) obtained in Example 1. The cellular injury was measured with the apparatus "TeraScan VPC, Minerva Tech K.K." which measured cellular injury by fluorescence microscopy. When a chemical compound having cellular cytotoxicity is allowed to act on cells which had been fluorescently labeled in advance, the cell membrane is destructed and the fluorescent pigment leaks. As a result, after the cellular injury, only cells remaining without suffering injury generate fluorescence. Based on the above-mentioned principle, the difference between the fluorescence strength before injury and the fluorescence strength after injury was determined with a cellular injury-measuring device, and cellular cytotoxicity-inhibitory activities of various peptides were calculated. That is, hydrogen peroxide was used as the chemical compound having cellular cytotoxicity, and allowed to act in a concentration of 58.8 mmol/L in a phosphate buffer (PBS). It should be noted that hydrogen peroxide is known to be a typical molecular species of oxygen radical, while the oxygen radical is also known to be one of factors involved in hepatocellular damage in a living body.

The cells were fluorescently labeled with calcein-AM in advance. Various peptides prepared in Example 2 were added to a 96-well microplate including hydrogen peroxide and the cells, both of which had been dispensed in advance, followed by reaction at 37°C for 100 minutes. The ratio of decrease in the fluorescence strength of the cells after the reaction was determined with the cellular injury-measuring device, and the ratio (%) of the cells which suffered injury was calculated. The results are shown in Table 1, as the ratio (%) of cells which suffered injury when the peptide was added at each concentration, wherein the ratio (%) of cells which suffered injury without the peptides is defined as 100.

**[Table 1-1]**

| Ratio (%) of the cells which suffered injury | | | | | | |
|---|---|---|---|---|---|---|
| | Peptides | Concentration of Peptide (*µ*g/ml) | | | | |
| | | 0 | 1 | 10 | 100 | 1, 000 |
| (1) | Arg-Pro-Lys-His-Pro-Ile-Lys-His-Gln Gly-Leu-Pro-Gln-Glu | 100 | 101 | 82 | 65 | 35 |
| (2) | His-Ile-Gln-Lys-Glu-Asp-Val-Pro-Ser-Glu-Arg-Tyr-Leu-Gly-Tyr-Leu-Glu-Gln-Leu-Leu-Arg-Leu-Lys-Lys-Tyr-Lys-Val-Pro-Gln-Leu | 100 | 98 | 93 | 63 | 31 |
| (3) | Ile-Asn-Pro-Ser-Lys-Glu-Asn | 100 | 98 | 94 | 64 | 49 |
| (4) | Gln-His-Gln-Lys-Ala-Met-Lys-Pro | 100 | 96 | 92 | 70 | 40 |
| (5) | Lys-Phe-Gln-Ser-Glu-Glu-Gln | 100 | 96 | 85 | 64 | 45 |
| (6) | Asp-Lys-Ile-His-Pro-Phe | 100 | 98 | 92 | 64 | 34 |
| (7) | Ile-Pro-Pro-Leu-Thr-Gln-Thr-Pro-Val-Val-Val-Pro-Pro-Phe-Leu-Gln-Pro-Glu-Val-Met-Gly-Val-Ser-Lys-Val-Lys-Glu-Ala-Met-Ala-Pro-Lys-Gln-Lys-Glu-Met | 100 | 95 | 92 | 68 | 40 |
| (8) | Tyr-Gln-Glu-Pro-Val-Leu-Gly-Pro-Val-Arg | 100 | 98 | 85 | 58 | 38 |
| (9) | pyroGlu-Glu-Lys-Asn-Met | 100 | 102 | 93 | 59 | 32 |
| (10) | Arg-Pro-Lys-His | 100 | 107 | 92 | 61 | 44 |
| (11) | Arg-Pro-Lys-His-Pro-Ile-Lys-His-Gln-Gly-Leu | 100 | 101 | 79 | 52 | 28 |
| (12) | His-Pro-Ile-Lys | 100 | 101 | 95 | 64 | 43 |
| (13) | His-Pro-Ile-Lys-His-Gln | 100 | 96 | 89 | 64 | 28 |
| (14) | His-Pro-Ile-Lys-His-Gln-Gly-Leu-Pro | 100 | 106 | 91 | 56 | 31 |
| (15) | His-Pro-Ile-Lys-His-Gln-Gly-Leu-Pro- | 100 | 96 | 92 | 58 | 35 |

**[Table 1-2]**

| | | | | | | |
|---|---|---|---|---|---|---|
| | Gln | | | | | |
| (16) | Val-Ala-Pro-Phe-Pro | 100 | 101 | 82 | 69 | 46 |
| (17) | Lys-Val-Asn | 100 | 101 | 81 | 67 | 50 |
| (18) | His-Ile-Gln-Lys-Glu-Asp-Val-Pro-Ser-Glu-Arg-Tyr | 100 | 98 | 85 | 56 | 39 |
| (19) | Asp-Val-Pro-Ser-Glu-Arg-Tyr-Leu-Gly-Tyr-Leu-Glu-Gln-Leu-Leu-Arg-Leu-Lys- Lys-Tyr-Lys-Val-Pro-Gln-Leu | 100 | 104 | 91 | 70 | 38 |
| (20) | Ile-Asn-Pro-Ser | 100 | 104 | 85 | 68 | 48 |
| (21) | Lys-Phe-Gln-Ser-Glu | 100 | 100 | 92 | 64 | 56 |
| (22) | Phe-Gln-Ser-Glu | 100 | 102 | 88 | 67 | 47 |
| (23) | Phe-Gln-Scr-Glu-Glu-Gln | 100 | 106 | 95 | 60 | 47 |
| (24) | Ile-Pro-Pro-Leu-Thr-Gln-Thr-Pro-Val-Val-Val | 100 | 106 | 87 | 63 | 51 |
| (25) | Leu-Thr-Gln-Thr-Pro-Val-Val-Val-Pro-Pro-Phe-Leu-Gln-Pro | 100 | 99 | 93 | 62 | 48 |
| (26) | Thr-Gln-Thr-Pro-Val-Val-Val-Pro-Pro-Phe-Leu-Gln-Pro-Glu-Val-Met | 100 | 107 | 81 | 55 | 38 |
| (27) | Gly-Val-Ser-Lys-Val-Lys-Glu-Ala-Met-Ala | 100 | 99 | 85 | 70 | 51 |
| (28) | Leu-Leu-Tyr-Gln-Glu-Pro-Val-Leu-Gly-Pro-Val-Arg-Gly-Pro-Phe-Pro-Ile | 100 | 97 | 85 | 67 | 46 |

As shown in Table 1, when each peptide was added, the ratio (%) which suffered cellular injury was reduced depending on the concentration of the peptide. Thus, the result of this Test Example confirmed that the peptide which includes any one of amino acid sequences represented by the formulae (1) to (28) had a hepatocellular damage-inhibitory activity, and was useful for the prevention and improvement of a hepatic dysfunction.

### EXAMPLE 2

Each of the ingredients as shown in Table 2 were mixed in accordance with the composition, filled into a container, followed by heat sterilization to manufacture a beverage for inhibiting hepatic dysfunction containing the peptide having the hepatic dysfunction-inhibitory effect of the present invention.

**[Table 2]**

| | | |
|---|---|---|
| Mixed isomerized sugar | 15.4 | (Weight %) |
| Fruit juice | 10.0 | |
| Citric acid | 0.5 | |
| Arg-Pro-Lys-His-Pro-Ile-Lys-His-Gln-Gly-Leu-Pro-Gln-Glu (EXAMPLE 1) | 0.1 | |
| Flavoring | 0.2 | |
| Water | 73.8 | |

### EXAMPLE 3

A dough having composition as shown in Table 3 was prepared, shaped, and then baked to manufacture a biscuit for inhibiting hepatic dysfunction containing the peptide having the hepatic dysfunction-inhibitory effect of the present invention.

**[Table 3]**

| | | |
|---|---|---|
| Wheat flour | 51.0 | (Weight %) |
| Sugar | 20.0 | |
| Table salt | 0.5 | |
| Margarine | 12.5 | |
| Egg | 12.5 | |
| Water | 2.5 | |
| Mineral mixture | 0.8 | |
| Gln-His-Gln-Lys-Ala-Met-Lys-Pro (EXAMPLE 1) | 0.2 | |

### EXAMPLE 4

Each of the ingredients as shown in Table 4 were mixed, in accordance with the composition, to manufacture a food for dogs containing the peptide having the hepatic dysfunction-inhibitory effect of the present invention.

**[Table 4]**

| | | |
|---|---|---|
| Soy bean cake | 12.0 | (Weight %) |
| Powdered skim milk | 14.9 | |
| Soybean oil | 4.0 | |
| Corn oil | 2.0 | |
| Palm oil | 28.0 | |
| Cornstarch | 15.0 | |
| Wheat flour | 8.0 | |
| Wheat bran | 2.0 | |
| Vitamin mixture | 9.0 | |
| Mineral mixture | 2.0 | |
| Cellulose | 3.0 | |
| pyroGlu-Glu-Lys-Asn-Met (EXAMPLE 1) | 0.1 | |

## Claims

1. A peptide which has an amino acid sequence represented by any one of the following formulae (1) to (28), provided that serine (Ser) is phosphorylated:
(1) Arg-Pro-Lys-His-Pro-Ile-Lys-His-Gln-Gly-Leu-Pro-Gln-Glu
(2) His-Ile-Gln-Lys-Glu-Asp-Val-Pro-Ser-Glu-Arg-Tyr-Leu-Gly-Tyr-Leu-Glu-Gln-Leu-Leu-Arg-Leu-Lys-Lys-Tyr-Lys-Val-Pro-Gln-Leu
(3) Ile-Asn-Pro-Ser-Lys-Glu-Ash
(4) Gln-His-Gln-Lys-Ala-Met-Lys-Pro
(5) Lys-Phe-Gln-Ser-Glu-Glu-Gln
(6) Asp-Lys-Ile-His-Pro-Phe
(7) Ile-Pro-Pro-Leu-Thr-Gln-Thr-Pro-Val-Val-Val-Pro-Pro-Phe-Leu-Gln-Pro-Glu-Val-Met-Gly-Val-Ser-Lys-Val-Lys-Glu-Ala-Met-Ala-Pro-Lys-Gln-Lys-Glu-Met
(8) Tyr-Gln-Glu-Pro-Val-Leu-Gly-Pro-Val-Arg
(9) pyroGlu-Glu-Lys-Asn-Met (pyroGlu: pyroglutamate)
(10) Arg-Pro-Lys-His
(11) Arg-Pro-Lys-His-Pro-Ile-Lys-His-Gln-Gly-Leu
(12) His-Pro-Ile-Lys
(13) His-Pro-Ile-Lys-His-Gln
(14) His-Pro-Ile-Lys-His-Gln-Gly-Leu-Pro
(15) His-Pro-Ile-Lys-His-Gln-Gly-Leu-Pro-Gln
(16) Val-Ala-Pro-Phe-Pro
(17) Lys-Val-Asn
(18) His-Ile-Gln-Lys-Glu-Asp-Val-Pro-Ser-Glu-Arg-Tyr
(19) Asp-Val-Pro-Ser-Glu-Arg-Tyr-Leu-Gly-Tyr-Leu-Glu-Gln-Leu-Leu-Arg-Leu-Lys-Lys-Tyr-Lys-Val-Pro-Gln-Leu
(20) Ile-Asn-Pro-Ser
(21) Lys-Phe-Gln-Ser-Glu
(22) Phe-Gln-Ser-Glu
(23) Phe-Gln-Ser-Glu-Glu-Gln
(24) Ile-Pro-Pro-Leu-Thr-Gln-Thr-Pro-Val-Val-Val
(25) Leu-Thr-Gln-Thr-Pro-Val-Val-Val-Pro-Pro-Phe-Leu-Gln-Pro
(26) Thr-Gln-Thr-Pro-Val-Val-Val-Pro-Pro-Phe-Leu-Gln-Pro-Glu-Val-Met
(27) Gly-Val-Ser-Lys-Val-Lys-Glu-Ala-Met-Ala
(28) Leu-Leu-Tyr-Gln-Glu-Pro-Val-Leu-Gly-Pro-Val-Arg-Gly-Pro-Phe-Pro-Ile for use in the treatment or prevention of a disease due to hepatic dysfunction wherein the disease is selected from the group consisting of acute hepatitis, chronic hepatitis, viral hepatitis, alcoholic fatty liver, hepatic cirrhosis, and liver cancer.

2. A peptide which has an amino acid sequence represented by any one of the following formulae (1) to (28), provided that serine (Ser) is phosphorylated:
(1) Arg-Pro-Lys-His-Pro-Ile-Lys-His-Gln-Gly-Leu-Pro-Gln-Glu
(2) His-Ile-Gln-Lys-Glu-Asp-Val-Pro-Ser-Glu-Arg-Tyr-Leu-Gly-Tyr-Leu-Glu-Gln-Leu-Leu-Arg-Leu-Lys-Lys-Tyr-Lys-Val-Pro-Gln-Leu
(3) Ile-Asn-Pro-Ser-Lys-Glu-Asn
(4) Gln-His-Gln-Lys-Ala-Met-Lys-Pro
(5) Lys-Phe-Gln-Ser-Glu-Glu-Gln
(6) Asp-Lys-Ile-His-Pro-Phe
(7) Ile-Pro-Pro-Leu-Thr-Gln-Thr-Pro-Val-Val-Val-Pro-Pro-Phe-Leu-Gln-Pro-Glu-Val-Met-Gly-Val-Ser-Lys-Val-Lys-Glu-Ala-Met-Ala-Pro-Lys-Gln-Lys-Glu-Met
(8) Tyr-Gln-Glu-Pro-Val-Leu-Gly-Pro-Val-Arg
(9) pyroGlu-Glu-Lys-Asn-Met (pyroGlu: pyroglutamate)
(10) Arg-Pro-Lys-His
(11) Arg-Pro-Lys-His-Pro-Ile-Lys-His-Gln-Gly-Leu
(12) His-Pro-Ile-Lys
(13) His-Pro-Ile-Lys-His-Gln
(14) His-Pro-Ile-Lys-His-Gln-Gly-Leu-Pro
(15) His-Pro-Ile-Lys-His-Gln-Gly-Leu-Pro-Gln
(16) Val-Ala-Pro-Phe-Pro
(17) Lys-Val-Asn
(18) His-Ile-Gln-Lys-Glu-Asp-Val-Pro-Ser-Glu-Arg-Tyr
(19) Asp-Val-Pro-Ser-Glu-Arg-Tyr-Leu-Gly-Tyr-Leu-Glu-Gln-Leu-Leu-Arg-Leu-Lys-Lys-Tyr-Lys-Val-Pro-Gln-Leu
(20) Ile-Asn-Pro-Ser
(21) Lys-Phe-Gln-Ser-Glu
(22) Phe-Gln-Ser-Glu
(23) Phe-Gln-Ser-Glu-Glu-Gln
(24) Ile-Pro-Pro-Leu-Thr-Gln-Thr-Pro-Val-Val-Val
(25) Leu-Thr-Gln-Thr-Pro-Val-Val-Val-Pro-Pro-Phe-Leu-Gln-Pro
(26) Thr-Gln-Thr-Pro-Val-Val-Val-Pro-Pro-Phe-Leu-Gln-Pro-Glu-Val-Met
(27) Gly-Val-Ser-Lys-Val-Lys-Glu-Ala-Met-Ala
(28) Leu-Leu-Tyr-Gln-Glu-Pro-Val-Leu-Gly-Pro-Val-Arg-Gly-Pro-Phe-Pro-Ile,
for use as a cytotoxicity-inhibitor.

3. The peptide according to claim 2 wherein the cytotoxicity-inhibitor is a cytotoxicity-inhibitor in hepatocytes.

4. The peptide according to claim 1 or 2, wherein the peptide is derived from milk protein.

5. The peptide according to any of the preceding claims, wherein said peptide is comprised in a food, beverage or feed.

## Patentansprüche

1. Peptid mit einer Aminosäuresequenz, die durch eine der nachstehend aufgeführten Formeln (1) bis (28) dargestellt ist, mit der Maßgabe, dass Serin (Ser) phosphoryliert ist:
(1) Arg-Pro-Lys-His-Pro-Ile-Lys-His-Gln-Gly-Leu-Pro-Gln-Glu
(2) His-Ile-Gln-Lys-Glu-Asp-Val-Pro-Ser-Glu-Arg-Tyr-Leu-Gly-Tyr-Leu-Glu-Gln-Leu-Leu-Arg-Leu-Lys-Lys-Tyr-Lys-Val-Pro-Gln-Leu
(3) Ile-Asn-Pro-Ser-Lys-Glu-Ash
(4) Gln-His-Gln-Lys-Ala-Met-Lys-Pro
(5) Lys-Phe-Gln-Ser-Glu-Glu-Gln
(6) Asp-Lys-Ile-His-Pro-Phe
(7) Ile-Pro-Pro-Leu-Thr-Pro-Val-Val-Val-Pro-Pro-Phe-Leu-Gln-Pro-Glu-Val-Met-Gly-Val-Ser-Lys-Val-Glu-Ala-Met-Ala-Pro-Lys-Gln-Lys-Glu-Met
(8) Tyr-Gln-Glu-Pro-Val-Leu-Gly-Pro-Val-arg
(9) pyroGlu-Glu-Lys-Asn-Met (pyroGlu: pyroglutamate)
(10) Arg-Pro-Lys-His
(11) Arg-Pro-Lys-His-Pro-Ile-Lys-His-Gln-Gly-Leu
(12) His-Pro-Ile-Lys
(13) His-Pro-Ile-Lys-His-Gln
(14) His-Pro-Ile-Lys-His-Gln-Gly-Leu-Pro
(15) His-Pro-Ile-Lys-His-Gln-Gly-Leu-Pro-Gln
(16) Val-Ala-Pro-Phe-Pro
(17) Lys-Val-Asn
(18) His-Ile-Gln-Lys-Glu-Asp-Val-Pro-Ser-Glu-Arg-Tyr
(19) Asp-Val-Pro-Ser-Glu-Arg-Tyr-Leu-Gly-Tyr-Leu-Glu-Gln-Leu-Leu-Arg-Leu-Lys-Lys-Tyr-Lys-Val-Pro-Gln-Leu
(20) Ile-Asn-Pro-Ser
(21) Lys-Phe-Gln-Ser-Glu
(22) Phe-Gln-Ser-Glu
(23) Phe-Gln-Ser-Glu-Glu-Gln
(24) Ile-Pro-Pro-Leu-Thr-Gln-Thr-Pro-Val-Val-Val
(25) Leu-Thr-Gln-Thr-Pro-Val-Val-Val-Pro-Pro-Phe-Leu-Gln-Pro
(26) Thr-Gln-Thr-Pro-Val-Val-Val-Pro-Pro-Phe-Leu-Gln-Pro-Glu-Val-Met
(27) Gly-Val-Ser-Lys-Val-Lys-Glu-Ala-Met-Ala
(28) Leu-Leu-Tyr-Gln-Glu-Pro-Val-Leu-Gly-Pro-Val-Arg-Gly-Pro-Phe-Pro-Ile,
zur Verwendung bei der Behandlung oder zur Vorbeugung einer Erkrankung aufgrund einer Leberstörung, worbei die Erkrankung ausgewählt ist aus der Gruppe bestehend aus akuter Hepatitis, chronischer Hepatitis, viraler Hepatitis, alkoholischer Fettleber, Leberzirrhose und Leberkrebs.

2. Peptid mit einer Aminosäuresequenz, die durch eine der nachstehend aufgeführten Formeln (1) bis (28) dargestellt ist, mit der Maßgabe, dass Serin (Ser) phosphoryliert ist:
(1) Arg-Pro-Lys-His-Pro-Ile-Lys-His-Gln-Gly-Leu-Pro-Gln-Glu
(2) His-Ile-Gln-Lys-Glu-Asp-Val-Pro-Ser-Glu-Arg-Tyr-Leu-Gly-Tyr-Leu-Glu-Gln-Leu-Leu-Arg-Leu-Lys-Lys-Tyr-Lys-Val-Pro-Gln-Leu
(3) Ile-Asn-Pro-Ser-Lys-Glu-Asn
(4) Gln-His-Gln-Lys-Ala-Met-Lys-Pro
(5) Lys-Phe-Gln-Ser-Glu-Glu-Glu
(6) Asp-Lys-Ile-His-Pro-Phe
(7) Ile-Pro-Pro-Leu-Thr-Gln-Thr-Pro-Val-Val-Val-Pro-Pro-Phe-Leu-Gln-Pro-Glu-Val-Met-Gly-Val-Ser-Lys-Val-Lys-Glu-Ala-Met-ALa-Pro-Lys-Gln-Lys-Glu-Met
(8) Tyr-Gln-Glu-Glu-Pro-Val-Leu-Gly-Pro-Val-Arg
(9) pyroGlu-Glu-Lys-Asn-Met (pyroGlu: pyroglutamate)
(10) Arg-Pro-Lys-His
(11) Arg-Pro-Lys-His-Pro-Ile-Lys-His-His-Gln-Gly-Leu
(12) His-Pro-Ile-Lys
(13) His-Pro-Ile-Lys-His-Gln
(14) His-Pro-Ile-Lys-His-Gln-Gly-Leu-Pro
(15) His-Pro-Ile-Lys-His-Gln-Gly-Leu-Pro-Gln
(16) Val-Ala-Pro-Phe-Pro
(17) Lys-Val-Asn
(18) His-Ile-Gln-Lys-Glu-Asp-Val-Pro-Ser-Glu-Arg-Tyr
(19) Asp-Val-Pro-Ser-Glu-Arg-Tyr-Leu-Gly-Tyr-Leu-Glu-Gln-Leu-Leu-Arg-Leu-Lys-Lys-Tyr-Lys-Val-Pro-Gln-Leu
(20) Ile-Asn-Pro-Ser
(21) Lys-Phe-Gln-Ser-Glu
(22) Phe-Gln-Ser-Glu
(23) Phe-Gln-Ser-Glu-Glu-Gln
(24) Ile-Pro-Pro-Leu-Thr-Gln-Thr-Pro-Val-Val-Val
(25) Leu-Thr-Gln-Thr-Pro-Val-Val-Val-Pro-Pro-Phe-Leu-Gln-Pro
(26) Thr-Gln-Thr-Pro-Val-Val-Val-Pro-Pro-Phe-Leu-Gln-Pro-Glu-Val-Met
(27) Gly-Val-Ser-Lys-Val-Lys-Glu-Ala-Met-Ala
(28) Leu-Leu-Tyr-Gln-Glu-Pro-Val-Leu-Gly-Pro-Val-Arg-Gly-Pro-Phe-Pro-Ile,
zur Verwendung als Cytotoxizitätsinhibitor.

3. Peptid nach Anspruch 2, worin der Cytotoxizitätsinhibitor ein Cytotoxizitätsinhibitor bei Hepatozyten ist.

4. Peptid nach Anspruch 1 oder 2, worin das Peptid von Milchprotein abgeleitet ist.

5. Peptid nach einem der vorstehenden Ansprüche, worin das Peptid in einem Nahrungsmittel, Getränk oder Futtermittel enthalten ist.

## Revendications

1. Peptide qui a une séquence d'acides aminés représentée par l'une quelconque des formules (1) à (28) suivantes, à condition que la sérine (Ser) soit phosphorylée :
(1) Arg-Pro-Lys-His-Pro-Ile-Lys-His-Gln-Gly-Leu-Pro-Gln-Glu
(2) His-Ile-Gln-Lys-Glu-Asp-Val-Pro-Ser-Glu-Arg-Tyr-Leu-Gly-Tyr-Leu-Glu-Gln-Leu-Leu-Arg-Leu-Lys-Lys-Tyr-Lys-Val-Pro-Gln-Leu
(3) Ile-Asn-Pro-Ser-Lys-Glu-Asn
(4) Gln-His-Gln-Lys-Ala-Met-Lys-Pro
(5) Lys-Phe-Gln-Ser-Glu-Glu-Gln
(6) Asp-Lys-Ile-His-Pro-Phe
(7) Ile-Pro-Pro-Leu-Thr-Gln-Thr-Pro-Val-Val-Val-Pro-Pro-Phe-Leu-Gln-Pro-Glu-Val-Met-Gly-Val-Ser-Lys-Val-Lys-Glu-Ala-Met-Ala-Pro-Lys-Gln-Lys-Glu-Met
(8) Tyr-Gln-Glu-Pro-Val-Leu-Gly-Pro-Val-Arg
(9) pyroGlu-Glu-Lys-Asn-Met (pyroGlu : pyroglutamate)
(10) Arg-Pro-Lys-His
(11) Arg-Pro-Lys-His-Pro-Ile-Lys-His-Gln-Gly-Leu
(12) His-Pro-Ile-Lys
(13) His-Pro-Ile-Lys-His-Gln
(14) His-Pro-Ile-Lys-His-Gln-Gly-Leu-Pro
(15) His-Pro-Ile-Lys-His-Gln-Gly-Leu-Pro-Gln
(16) Val-Ala-Pro-Phe-Pro
(17) Lys-Val-Asn
(18) His-Ile-Gln-Lys-Glu-Asp-Val-Pro-Ser-Glu-Arg-Tyr
(19) Asp-Val-Pro-Ser-Glu-Arg-Tyr-Leu-Gly-Tyr-Leu-Glu-Gln-Leu-Leu-Arg-Leu-Lys-Lys-Tyr-Lys-Val-Pro-Gln-Leu
(20) Ile-Asn-Pro-Ser
(21) Lys-Phe-Gln-Ser-Glu
(22) Phe-Gln-Ser-Glu
(23) Phe-Gln-Ser-Glu-Glu-Gln
(24) Ile-Pro-Pro-Leu-Thr-Gln-Thr-Pro-Val-Val-Val
(25) Leu-Thr-Gln-Thr-Pro-Val-Val-Val-Pro-Pro-Phe-Leu-Gln-Pro
(26) Thr-Gln-Thr-Pro-Val-Val-Val-Pro-Pro-Phe-Leu-Gln-Pro-Glu-Val-Met
(27) Gly-Val-Ser-Lys-Val-Lys-Glu-Ala-Met-Ala
(28) Leu-Leu-Tyr-Gln-Glu-Pro-Val-Leu-Gly-Pro-Val-Arg-Gly-Pro-Phe-Pro-Ile,
pour une utilisation dans le traitement ou la prévention d'une maladie due à un dysfonctionnement hépatique, où la maladie est choisie dans le groupe constitué par une hépatite aiguë, une hépatite chronique, une hépatite virale, une stéatose hépatique alcoolique, une cirrhose hépatique, et un cancer du foie.

2. Peptide qui a une séquence d'acides aminés représentée par l'une quelconque des formules (1) à (28) suivantes, à condition que la sérine (Ser) soit phosphorylée :
(1) Arg-Pro-Lys-His-Pro-Ile-Lys-His-Gln-Gly-Leu-Pro-Gln-Glu
(2) His-Ile-Gln-Lys-Glu-Asp-Val-Pro-Ser-Glu-Arg-Tyr-Leu-Gly-Tyr-Leu-Glu-Gln-Leu-Leu-Arg-Leu-Lys-Lys-Tyr-Lys-Val-Pro-Gln-Leu
(3) Ile-Asn-Pro-Ser-Lys-Glu-Asn
(4) Gln-His-Gln-Lys-Ala-Met-Lys-Pro
(5) Lys-Phe-Gln-Ser-Glu-Glu-Gln
(6) Asp-Lys-Ile-His-Pro-Phe
(7) Ile-Pro-Pro-Leu-Thr-Gln-Thr-Pro-Val-Val-Val-Pro-Pro-Phe-Leu-Gln-Pro-Glu-Val-Met-Gly-Val-Ser-Lys-Val-Lys-Glu-Ala-Met-Ala-Pro-Lys-Gln-Lys-Glu-Met
(8) Tyr-Gln-Glu-Pro-Val-Leu-Gly-Pro-Val-Arg
(9) pyroGlu-Glu-Lys-Asn-Met (pyroGlu : pyroglutamate)
(10) Arg-Pro-Lys-His
(11) Arg-Pro-Lys-His-Pro-Ile-Lys-His-Gln-Gly-Leu
(12) His-Pro-Ile-Lys
(13) His-Pro-Ile-Lys-His-Gln
(14) His-Pro-Ile-Lys-His-Gln-Gly-Leu-Pro
(15) His-Pro-Ile-Lys-His-Gln-Gly-Leu-Pro-Gln
(16) Val-Ala-Pro-Phe-Pro
(17) Lys-Val-Asn
(18) His-Ile-Gln-Lys-Glu-Asp-Val-Pro-Ser-Glu-Arg-Tyr
(19) Asp-Val-Pro-Ser-Glu-Arg-Tyr-Leu-Gly-Tyr-Leu-Glu-Gln-Leu-Leu-Arg-Leu-Lys-Lys-Tyr-Lys-Val-Pro-Gln-Leu
(20) Ile-Asn-Pro-Ser
(21) Lys-Phe-Gln-Ser-Glu
(22) Phe-Gln-Ser-Glu
(23) Phe-Gln-Ser-Glu-Glu-Gln
(24) Ile-Pro-Pro-Leu-Thr-Gln-Thr-Pro-Val-Val-Val
(25) Leu-Thr-Gln-Thr-Pro-Val-Val-Val-Pro-Pro-Phe-Leu-Gln-Pro
(26) Thr-Gln-Thr-Pro-Val-Val-Val-Pro-Pro-Phe-Leu-Gln-Pro-Glu-Val-Met
(27) Gly-Val-Ser-Lys-Val-Lys-Glu-Ala-Met-Ala
(28) Leu-Leu-Tyr-Gln-Glu-Pro-Val-Leu-Gly-Pro-Val-Arg-Gly-Pro-Phe-Pro-Ile,
pour une utilisation en tant qu'inhibiteur de la cytotoxicité.

3. Peptide selon la revendication 2, où l'inhibiteur de la cytotoxicité est un inhibiteur de la cytotoxicité dans les hépatocytes.

4. Peptide selon la revendication 1 ou 2, où le peptide est dérivé des protéines du lait.

5. Peptide selon l'une quelconque des revendications précédentes, où ledit peptide est compris dans un aliment, une boisson ou un aliment pour animaux.


### SEQUENCE LISTING

<110>Snow Brand Milk Products Co.,Ltd.
<120>Peptide
<160>28
<210>1
   <211>14
   <212>PRT
   <213>Bovine
<400>
   Arg Pro Lys His Pro Ile Lys His Gln Gly Leu Pro Gln Glu
   1               5                   10
<210>2
   <211>30
   <212>PRT
   <213>Bovine
<400>
   His Ile Gln Lys Glu Asp Val Pro Ser Glu Arg Tyr Leu Gly Tyr Leu
   1               5                   10                  15
   Glu Gln Leu Leu Arg Leu Lys Lys Tyr Lys Val Pro Gln Leu
               20                  25
<210>3
   <211>7
   <212>PRT
   <213>Bovine
<400>
   Ile Asn Pro Ser Lys Glu Asn
   1               5
<210>4
   <211>8
   <212>PRT
   <213>Bovine
<400>
   Gln His Gln Lys Ala Met Lys Pro
   1               5
<210>5
   <211>7
   <212>PRT
   <213>Bovine
<400>
   Lys Phe Gln Ser Glu Glu Gln
   1               5
<210>6
   <211>6
   <212>PRT
   <213>Bovine
<400>
   Asp Lys Ile His Pro Phe
   1               5
<210>7
   <211>36
   <212>PRT
   <213>Bovine
<400>
   Ile Pro Pro Leu Thr Gln Thr Pro Val Val Val Pro Pro Phe Leu Gln
   1               5                   10                  15
   Pro Glu Val Met Gly Val Ser Lys Val Lys Glu Ala Met Ala Pro Lys
               20                  25                  30
   Gln Lys Glu Met
           35
<210>8
   <211>10
   <212>PRT
   <213>Bovine
<400>
   Tyr Gln Glu Pro Val Leu Gly Pro Val Arg
   1               5                   10
<210>9
   <211>5
   <212>PRT
   <213>Bovine
<220>
   <221>PYRROLIDONE CARBOXYLIC ACID
   <222>
   <223>
<400>
   Xaa Glu Lys Asn Met
   1 5
<210>10
   <211>4
   <212>PRT
   <213>Bovine
<400>
   Arg Pro Lys His
   1
<210>11
   <211>11
   <212>PRT
   <213>Bovine
<400>
   Arg Pro Lys His Pro Ile Lys His Gln Gly Leu
   1 5 10
<210>12
   <211>4
   <212>PRT
   <213>Bovine
<400>
   His Pro Ile Lys
   1
<210>13
   <211>6
   <212>PRT
   <213>Bovine
<400>
   His Pro Ile Lys His Gln
   1 5
<210>14
   <211>9
   <212>PRT
   <213>Bovine
<400>
   His Pro Ile Lys His Gln Gly Leu Pro
   1 5
<210>15
   <211>10
   <212>PRT
   <213>Bovine
<400>
   His Pro Ile Lys His Gln Gly Leu Pro Gln
   1 5 10
<210>16
   <211>5
   <212>PRT
   <213>Bovine
<400>
   Val Ala Pro Phe Pro
   1 5
<210>17
   <211>3
   <212>PRT
   <213>Bovine
<400>
   Lys Val Asn
   1
<210>18
   <211>12
   <212>PRT
   <213>Bovine
<400>
   His Ile Gln Lys Glu Asp Val Pro Ser Glu Arg Tyr
   1 5 10
<210>19
   <211>25
   <212>PRT
   <213>Bovine
<400>
   Asp Val Pro Ser Glu Arg Tyr Leu Gly Tyr Leu Glu Gln Leu Leu Arg
   1 5 10 15
   Leu Lys Lys Tyr Lys Val Pro Gln Leu
   20 25
<210>20
   <211>4
   <212>PRT
   <213>Bovine
<400>
   Ile Asn Pro Ser
   1
<210>21
   <211>5
   <212>PRT
   <213>Bovine
<400>
   Lys Phe Gln Ser Glu
   1 5
<210>22
   <211>4
   <212>PRT
   <213>Bovine
<400>
   Phe Gln Ser Glu
   1
<210>23
   <211>6
   <212>PRT
   <213>Bovine
<400>
   Phe Gln Ser Glu Glu Gln
   1 5
<210>24
   <211>11
   <212>PRT
   <213>Bovine
<400>
   Ile Pro Pro Leu Thr Gln Thr Pro Val Val Val
   1 5 10
<210>25
   <211>14
   <212>PRT
   <213>Bovine
<400>
   Leu Thr Gln Thr Pro Val Val Val Pro Pro Phe Leu Gln Pro
   1 5 10
<210>26
   <211>16
   <212>PRT
   <213>Bovine
<400>
   Thr Gln Thr Pro Val Val Val Pro Pro Phe Leu Gln Pro Glu Val Met
   1 5 10 15
<210>27
   <211>10
   <212>PRT
   <213>Bovine
<400>
   Gly Val Ser Lys Val Lys Glu Ala Met Ala
   1 5 10
<210>28
   <211>17
   <212>PRT
   <213>Bovine
<400>
   Leu Leu Tyr Gln Glu Pro Val Leu Gly Pro Val Arg Gly Pro Phe Pro
   1 5 10 15
   Ile